# EUROPEAN PATENT SPECIFICATION

(11) **EP 2 820 415 B1**
(45) Date of publication and mention of the grant of the patent: **26.07.2017**
(21) Application number: 13703492.2
(22) Date of filing: 25.01.2013
(51) Int. Cl.: G01N 33/50

(54) **NEUROGENESIS SCREENING METHOD AND SYSTEM USING ADIPOSE TISSUE DERIVED STEM CELLS**
NEUROGENESE-SCREENINGVERFAHREN UND -SYSTEM UNTER VERWENDUNG VON AUS FETTGEWEBE STAMMENDEN STAMMZELLEN
PROCÉDÉ ET SYSTÈME DE CRIBLAGE POUR LA NEUROGENÈSE FAISANT APPEL À DES CELLULES SOUCHES DÉRIVÉES DU TISSU ADIPEUX

(30) Priority: 29.02.2012 US 201213408485
(43) Date of publication of application: 07.01.2015
(73) Proprietor: MJN U.S. Holdings, LLC, Glenview, IL 60026 (US)
(72) Inventor: KUANG, Chenzhong, Newburgh Indiana 47630 (US); XIAO, Yan, Newburgh Indiana 47630 (US); JOUNI, Zeina, Battle Creek Michigan 79017 (US); POELS, Eduard, K., Baltimore Maryland 21211 (US); HONDMANN, Dirk, Winnetka Illinois 60093 (US)
(74) Representative: V.O.
(86) International application number: PCT/US2013/023094
(87) International publication number: WO 2013/130196

(56) References cited:
- WO-A1-2011/050476
- JP-A- 2010 130 968
- ASHJIAN PETER H ET AL: "In vitro differentiation of human processed lipoaspirate cells into early neural progenitors", PLASTIC AND RECONSTRUCTIVE SURGERY, WILLIAMS AND WILKINS CO., BALTIMORE, MD, US, vol. 111, no. 6, 1 May 2003 (2003-05-01), pages 1922-1931, XP008160657, ISSN: 0032-1052, DOI: 10.1097/01.PRS.0000055043.62589.05
- TINGTING HUANG ET AL: "Neuron-like Differentiation of Adipose-Derived Stem Cells From Infant Piglets in Vitro", J SPINAL CORD MED, vol. 30, 1 August 2007 (2007-08-01), pages S35-S40, XP055055932,
- SAFFORD KRISTINE M ET AL: "Neurogenic differentiation of murine and human adipose-derived stromal cells", BIOCHEMICAL AND BIOPHYSICAL RESEARCH COMMUNICATIONS, ACADEMIC PRESS INC. ORLANDO, FL, US, vol. 294, no. 2, 7 June 2002 (2002-06-07), pages 371-379, XP002384763, ISSN: 0006-291X, DOI: 10.1016/S0006-291X(02)00469-2
- Alan: "Human/Mouse/Rat Neural Lineage Functional Identification Kit", , 22 December 2011 (2011-12-22), XP055055953, Retrieved from the Internet: URL:http://www.funakoshi.co.jp/data/datash eet/RSD/SC028.pdf [retrieved on 2013-03-11]
- KAWAKITA E ET AL: "Docosahexaenoic acid promotes neurogenesis in vitro and in vivo", NEUROSCIENCE, NEW YORK, NY, US, vol. 139, no. 3, 1 January 2006 (2006-01-01), pages 991-997, XP024986670, ISSN: 0306-4522, DOI: 10.1016/J.NEUROSCIENCE.2006.01.021 [retrieved on 2006-01-01]

## Description

### TECHNICAL FIELD

The present disclosure relates to methods for identifying neurogenesis-modulating compounds, e.g., compounds that either promote or inhibit neurogenesis. More specifically, the disclosure relates to methods for identifying neurogenesis-modulating compounds using adipose-derived stem cells (ADSCs), and more particularly, human adipose-derived stem cells (hADSCs). The present invention is defined by the appended claims. Any subject-matter identified in the application as "invention", "embodiment", "aspect", etc., which exceeds the scope of the invention as represented by the claims does not form part of the claimed invention but only serves as background information to better understand the invention.

### BACKGROUND ART

Brain nutrients have become increasingly important additives in the diets of infants, children and pregnant and lactating women because of their ability to promote early brain development. Additionally, compounds useful for treating neurodegenerative disease or brain injury are continuously being sought. Neuro-toxic compounds, such as environmental, industrial or dietary toxins, need to be identified in order to remove or reduce exposure to such compounds. Methods for discovering such nutrients and toxins are often extremely time consuming and inefficient. Accordingly, there is a need to provide a reliable, consistent, and fast method for identifying compounds having neurological development benefits. Additionally, there is need to identify compounds that are neurologically harmful.

It has been demonstrated that stem cells, such as adipose-derived stem cells (ADSCs), can be differentiated into multiple mature cell phenotypes, including neuronal cells, in a reproducible manner (WO2011/050476). In particular, this has been demonstrated in human adipose-derived stem cells (hADSCs). hADSCs are a particularly useful research tool because they are readily available from commercial resources or liposuction procedures, and they do not involve the same potential controversies that arise from the use of embryonic stem cells. Furthermore, hADSCs are easily obtained from an individual patient, thus providing an opportunity for personalized medicine.

### DISCLOSURE OF THE INVENTION

One aspect of the present disclosure provides methods for identifying a neurogenesis-modulating compound using ADSCs. The methods are useful for identifying potential brain nutrients that may be used to supplement the diets of infants, children, and pregnant and lactating women. The present methods also are useful for identifying potential drug candidates for the treatment of neurological diseases and neurological injuries. Finally, the present methods are useful for identifying compounds that may be neurotoxic, for example compounds that are harmful to neurological development in fetuses, infants and children. Neurotoxic compounds also may contribute to neurologic diseases or may interfere with the treatment and healing of neurological diseases and injury.

Thus, in certain embodiments, the present disclosure provides a method for identifying a neurogenesis-modulating compound, comprising: culturing adipose-derived stem cells (ADSCs) in the presence of a candidate compound; and determining the extent of neurogenesis in the ADSCs. The aforementioned method may further comprise culturing ADSCs in the absence of the candidate compound, determining the extent of neurogenesis in the ADSCs cultured in the absence of the candidate compound, and comparing the extent of neurogenesis in the ADSCs cultured in the presence of the candidate compound to the extent of neurogenesis of the ADSCs cultured in the absence of the candidate compound. In some embodiments, the adipose-derived stem cells are human adipose-derived stem cells (hADSCs).

Without being bound by any particular theory, it is believed that an increase in the extent of neurogenesis in the ADSCs cultured in the presence of the candidate compound compared to the extent of neurogenesis in the ADSCs cultured in the absence of the candidate compound indicates that the candidate compound is a neurogenesis-promoting compound. On the other hand, a decrease in the extent of neurogenesis in the ADSCs cultured in the presence of the candidate compound compared to the extent of neurogenesis in the ADSCs cultured in the absence of the candidate compound indicates that the candidate compound is a neurogenesis-inhibiting compound.

In certain embodiments, the method further comprises culturing ADSCs in the presence of a known neurogenesis-promoting compound, such as docosahexaenoic acid (DHA), determining the extent of neurogenesis of the ADSCs cultured in the presence of DHA, and comparing the extent of neurogenesis in the ADSCs cultured in the presence of the candidate compound to the extent of neurogenesis in the ADSCs cultured in the presence of DHA, wherein an increase in the extent of neurogenesis in the ADSCs cultured in the presence of the candidate compound compared to the extent of neurogenesis in the ADSCs cultured in the presence of DHA indicates that the candidate compound is a neurogenesis-promoting compound.

In certain embodiments, the extent of neurogenesis is determined by observing a change in cell morphology of the ADSCs. The change in cell morphology includes, without limitation, shrinkage of cell cytoplasm, formation of a neurite, formation a dendrite-like projection, formation of an axon, or any combination thereof. Changes in cell morphology can be determined by any method for cellular analysis or visualization. For example, the change in cell morphology can be observed by microscopy, such as phase contrast microscopy. In other embodiments, the extent of neurogenesis is determined by observing cellular biomarkers indicative of neurogenesis.

In any of the aforementioned methods, the ADSCs are cultured in the presence of the candidate compound for a period of time sufficient for neurogenesis to occur, for example about 1 to about 5 days. Furthermore, the ADSCs may be cultured at an elevated temperature, such as from about 25 to about 45 °C.

The cultureware used for culturing the ADSCs may comprises a coating that promotes or supports neurogenesis, such as a coating that mimics the environment of the central nervous. For example, the cultureware may comprise a coating comprising poly-L-ornithine and bovine fibronectin.

The medium used to culture the ADSCs, in some embodiments, promotes or supports neurogenesis. For example, the medium may comprise a neural basal medium, epidermal growth factor (EGF), basic fibroblast growth factor (b-FGF), N2 supplement, and L-glutamine.

In certain embodiments, the method comprises priming the ADSCs for about 1 to about 5 days in a priming medium prior to culturing the cells in the presence of the candidate compound. The priming medium may comprise a neural basal medium, EGF, b-FGF, and N2 supplement. After priming, the ADSCs may be cultured in a medium comprising MesenPRO complete and the candidate compound for about 1 to about 5 days.

Another aspect of the present disclosure provides a method of promoting neurogenesis in ADSCs, comprising: culturing the ADSCs in the presence of a neurogenesis promoting compound. In certain embodiments, the method further comprises determining the extent of neurogenesis in the ADSCs.

Still another aspect of the disclosure relates to a system for identifying a neurogenesis-modulating compound, comprising: ADSCs; cultureware comprising a coating that mimics the central nervous system; and a culture medium for promoting neurogenesis. The coating for the cultureware may comprise, for example, bovine fibronectin and poly-L-ornithine. The culture medium may comprise a neural basal medium, EGF, b-FGF, N2 supplement, and L-glutamine. In other embodiments, the system comprises: ADSCs, cultureware comprising a coating that mimics the central nervous system, a priming medium, and a culture medium. The priming medium may comprise a neural basal medium, EGF, b-FGF, and N2supplement, while the culture medium may comprise MesenPRO Complete.

### BRIEF DESCRIPTION OF THE DRAWINGS

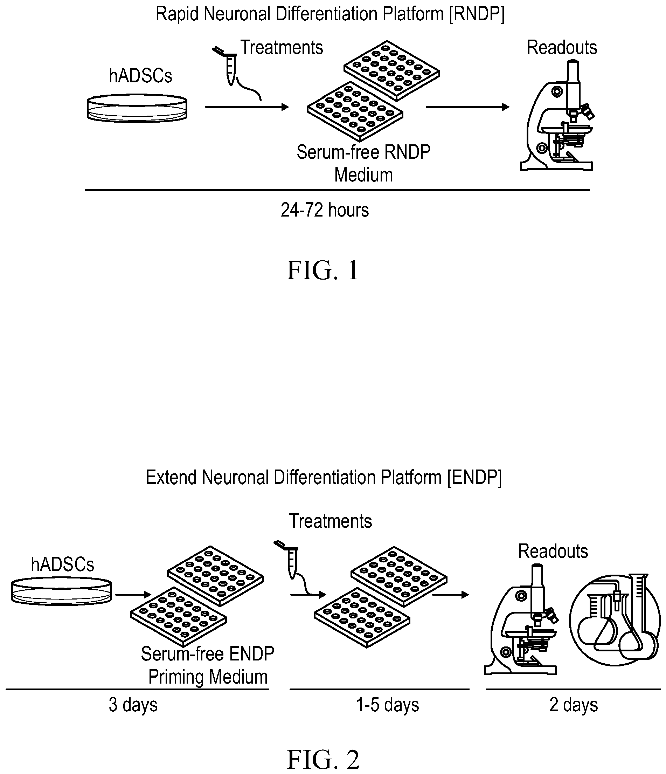

**Fig. 1** is a diagram depicting a rapid neuronal differentiation platform (RNDP) according to an embodiment of the present disclosure. hADSCs are cultured in a suitable culture medium and an appropriate amount a candidate compound (treatment) for 24-72 hours. The hADSCs are then evaluated to determining the extent of neurogenesis.
**Fig. 2** is a diagram depicting an extended neuronal differentiation platform (ENDP) according to an embodiment of the present disclosure. hADSCs are cultured in a suitable priming medium for up to three days. The priming medium is then replaced with a suitable culture medium (differentiation medium) and an appropriate amount of a candidate compound and cultured for 1 to 5 days. The hADSCs are then evaluated to determining the extent of neurogenesis.

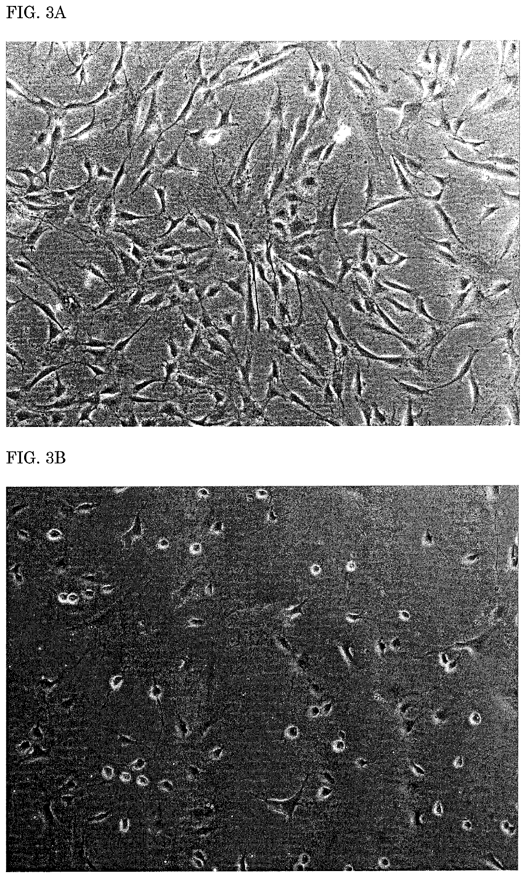

**FIG. 3A** depicts a phase contrast image of a ADSC's in a control experiment. **FIG. 3B** depicts a phase contrast image of ADSC's post brain nutrient treatment.

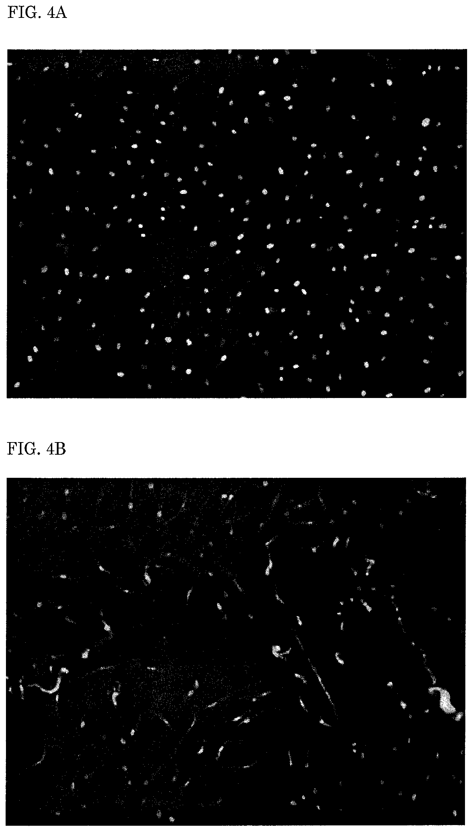

**FIG. 4A** is a control image from a cellular expression study in which cells are stained with an antibody against microtubule-associated protein 2 (MAP2), a neuronal marker. **FIG. 4B** is a post brain nutrient treatment image in the MAP2 expression study. Red fluorescence (indicated by the white streaks) demonstrates the expression of MAP2.

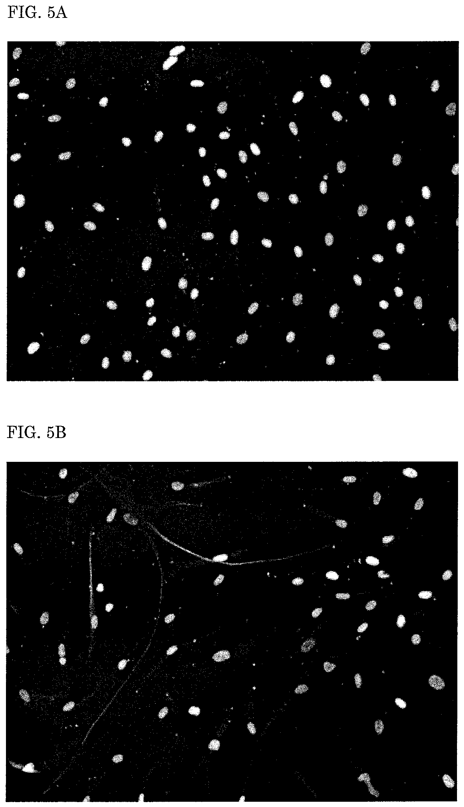

**FIG. 5A** is a control image from a cellular expression study in which cells are stained with an antibody against nestin, a neuronal marker. **FIG. 5B** is a post brain nutrient treatment image in the nestin expression study. Red fluorescence (indicated by the white streaks) demonstrates the expression of nestin.

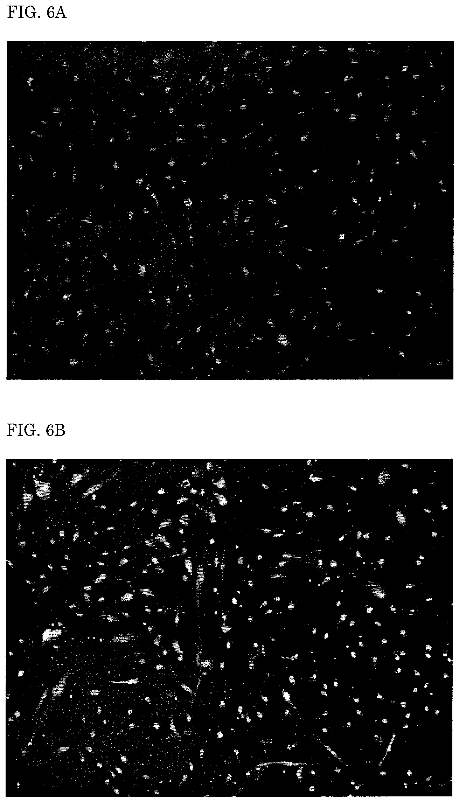

**FIG. 6A** is a control image from a cellular expression study in which cells are stained with an antibody against glial fibrillary acidic protein (GFAP), a neuronal marker. **FIG. 6B** is a post brain nutrient treatment image in the GFAP expression study. Red fluorescence (indicated by the white streaks) demonstrates the expression of GFAP.

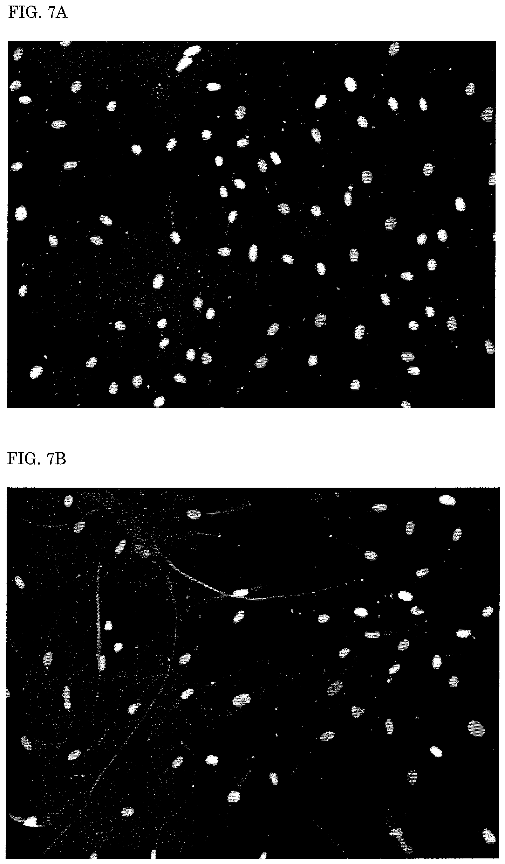

**FIG. 7A** is a control image from a. cellular expression study in which cells are stained with an antibody against beta III tubulin, a neuronal marker. **FIG. 7B** is a post brain nutrient treatment image in the beta III tubulin expression study. Red fluorescence (indicated by the white streaks) demonstrates the expression of beta III tubulin.

### BEST MODE FOR CARRYING OUT THE INVENTION

The present disclosure provides methods for identifying a neurogenesis-modulating compound comprising: culturing adipose-derived stem cells (ADSCs) in the presence of a candidate compound, and determining the extent of neurogenesis in the ADSCs.

"Neurogenesis" refers to the differentiation, generation or proliferation of neural cells from stem or progenitor cells *in vitro* or *in vivo*. The extent of neurogenesis can be determined by a variety of techniques known in the art, such as by observing morphological changes in the cells. Any method for cellular analysis or visualization is suitable for use in the present methods. For example, morphological changes in the ADSCs may be observed using a microscopic technique, such as phase contrast microscopy. Morphological changes that indicate neurogenesis include, but are not limited to, shrinkage of cytoplasm and the presence of neurites, axons and dendrites. In other embodiments, the extent of neurogenesis is determined by observing cellular biomarkers indicative of neurogenesis, such as by using biomarker expression experiments. Examples of such biomarkers include, but are not limited to, proteins such as neurofilaments, myelin basic protein, microtubule associated protein 2 (MAP2), nestin, □-III tubulin, glial fibrillar acidic protein (GFAP), S100 (a calcium binding protein), CNPase and GABA receptor.

A "neurogenesis-modulating compound" refers to a compound that affects neurogenesis, either by promoting or inhibiting neurogenesis. Thus, in some embodiments, neurogenesis-modulating compounds promote neurogenesis ("neurogenesis-promoting compounds"), while in other embodiments, the neurogenesis-modulating compounds inhibit or reduce neurogenesis ("neurogenesis-inhibiting compounds"). Compounds identified as promoting neurogenesis may advantageously be used as supplements in the diets of infants, children, and pregnant and lactating mothers in order to promote and support early brain development. These compounds also may be useful in treating neurodegenerative diseases or neurological injuries. Compounds identified as inhibiting neurogenesis may be potential toxins to be avoided or removed from the diets and environments of infants, children, and pregnant and lactating women. These compounds also may interfere with the treatment or healing of neurological diseases or injuries. Thus, neurogenesis-inhibiting compounds may also be avoided in the diets and environments of individuals suffering from neurological disease or injury.

A "candidate compound" refers to any compound to be tested for neurogenesis-modulating properties using the methods described herein. The candidate compounds include, without limitation, naturally occurring substances, synthetic compounds, or extracts, such as extracts of plant or animal tissues, fungi or bacteria. The candidate compound may be tested singly or it may be tested in combination with other candidate compounds or known neurogenesis-modulating compound in order to observe synergistic effects or to achieve higher throughput screening of compounds.

In certain embodiments, the method further comprises providing a negative control culture of ADSCs for comparison to the candidate compound. Accordingly, the method further comprises culturing ADSCs in the absence of the candidate compound, determining the extent of neurogenesis in the ADSCs cultured in the absence of the candidate compound, and comparing the extent of neurogenesis in the ADSCs cultured in the presence of the candidate compound to the extent of neurogenesis of the ADSCs cultured in the absence of the candidate compound. An increase in the extent of neurogenesis in the ADSCs cultured in the presence of the candidate compound compared to the extent of neurogenesis in the ADSCs cultured in the absence of the candidate compound indicates that the candidate compound is a neurogenesis-promoting compound. On the other hand, a decrease in the extent of neurogenesis in the ADSCs cultured in the presence of the candidate compound compared to the extent of neurogenesis in the ADSCs cultured in the absence of the candidate compound indicates that the candidate compound is a neurogenesis-inhibiting compound. The, the negative control culture provides additional information regarding the neurogenesis modulating properties of the candidate compounds.

In other embodiments, the method further comprises providing a positive control culture. Thus, the method further comprises culturing ADSCs in the presence a known neurogenesis-promoting compound, and determining the extent of neurogenesis in the ADSCs cultured in the presence of the neurogenesis-promoting compound. For example, DHA is known to promote early brain development and may be used as a positive control. Accordingly, the method may further comprise culturing ADSCs in the presence of DHA. An increase in the extent of neurogenesis in the ADSCs cultured in the presence of the candidate compound compared to the extent of neurogenesis in the ADSCs cultured in the presence of DHA indicates that the candidate compound is a superior neurogenesis-promoting compound than DHA.

During neurogenesis, the ADSCs may differentiate into neuronal cells, precursors to neuronal cells, and cells having neuronal properties. Accordingly, the extent of neurogenesis can be determined by observing morphological changes in the cells. Changes in cell morphology that are indicative of neurogenesis include, but are not limited to, shrinkage of cell cytoplasm, formation of a neurite, formation of a dendrite-like projection, formation of an axon, or a combination thereof. Other changes in cell morphology indicative of neurogenesis include development of a morphology that resembles bi-polar, tri-polar and multi-polar neuronal cells.

The aforementioned changes in cell morphology can be observed by a microscopic technique, such as by phase contrast microscopy. Phase contrast microscopy images of the ADSCs may be multiple times during the culturing of the ADSCs. For example, images may be taken prior to culturing with the candidate compound, and one or more times after addition of the candidate compound, such as three hours after, and then once daily thereafter.

The extent of neurogenesis can further be determined by measuring the percentage of ADSCs exhibiting neuronal differentiation and the length of cytoplasmic projections in the cells, such as neurites, axons and dendrites. The percentage of ADSCs exhibiting neuronal differentiation and length of cytoplasmic projections can be measured using Image J open software with an appropriate plug-in.

Changes in cellular biomarkers occur during neurogenesis. Thus, in some embodiments, a cellular expression study for neuronal markers is used to determine the extent of neurogenesis. Examples of such biomarkers include, but are not limited to, proteins such as neurofilaments, myelin basic protein, nestin, □-III tubulin, glial fibrillar acidic protein (GFAP), S100 (a calcium binding protein), microtubule associated protein 2 (MAP2), CNPase and GABA receptor. Additional techniques for determining neuronal differentiation include immunohisotlogical staining for neuronal markers, neuronal excitability measurements and western blotting for the expression of neural proteins.

In some embodiments, the ADSCs are human adipose-derived stem cells (hADSCs). hADSCs can advantageously be maintained in culture and readily passaged to provide multiple sub-cultures. Furthermore, hADSCs are readily available because they can be isolated from human adipose tissue collected during routine liposuction procedures and cryopreserved. hADSCs have the additional advantage of being readily obtained from an individual patient. The hADSCs thus obtained can be used in the methods described herein to screen a candidate compound for individualized use. Accordingly, personalized and optimized nutrition, drug treatment, or determination of sensitivity to neurotoxins can be achieved using the methods of the present disclosure.

The ADSCs may be cultured for a sufficient amount of time for neurogenesis to occur. Neurogenesis may be observed at varying times, depending on the brain nutrient tested. Thus, in some embodiments, neurogenesis may be observed after a few hours of culturing while in other embodiments, neurogenesis may be observed after several days of culturing. For example, the ADSCs may be cultured for about 1 hour to about 5 days, about 1 hour to about 3 days, about 3 hours to about 36 hours, about 12 hours to about 24 hours, or about 24 to about 36 hours. Furthermore, culturing of ADSC's may be continued for one, two, three or four weeks in order to achieve a more complete neuronal differentiation. The culturing of the ADSCs may further be performed at an elevated temperature, such as a temperature above room temperature. Such temperatures include about 25 to about 45 °C, about 30 to about 40 °C, or about 37 °C.

In the aforementioned methods, the ADSCs may advantageously be cultured in a medium that supports or promotes neurogenesis, for example by guiding the ADSCs to differentiate into neuronal cells. In some embodiments, the medium comprises a neural basal medium, epidermal growth factor (EGF), basic□fibroblast growth factor b-FGF, N2 supplement and L-glutamine. The ingredients for the culture medium are available from commercial sources. For example, the neural basal medium can be Neurobasal^{™} Medium, which is available from Invitrogen. Neural Basal Medium™ may include the ingredients listed in Table 1:

**Table 1: Neurobasal^{™} Medium**

| **Components** | **Molecular Weight** | **Concentration (mg/L)** | **mM** |
|---|---|---|---|
| **Amino Acids** | | | |
| Glycine | 75 | 30 | 0.4 |
| L-Alanine | 89 | 2 | 0.0225 |
| L-Arginine hydrochloride | 211 | 84 | 0.398 |
| L-Asparagine-H2O | 150 | 0.83 | 0.00553 |
| L-Cysteine | 121 | 31.5 | 0.26 |
| L-Histidine hydrochloride-H2O | 210 | 42 | 0.2 |
| L-Isoleucine | 131 | 105 | 0.802 |
| L-Leucine | 131 | 105 | 0.802 |
| L-Lysine hydrochloride | 183 | 146 | 0.798 |
| L-Methionine | 149 | 30 | 0.201 |
| L-Phenylalanine | 165 | 66 | 0.4 |
| L-Proline | 115 | 7.76 | 0.0675 |
| L-Serine | 105 | 42 | 0.4 |
| L-Threonine | 119 | 95 | 0.798 |
| L-Tryptophan | 204 | 16 | 0.0784 |
| L-Tyrosine | 181 | 72 | 0.398 |
| L-Valine | 117 | 94 | 0.803 |

| **Vitamins** | | | |
|---|---|---|---|
| Choline chloride | 140 | 4 | 0.0286 |
| D-Calcium pantothenate | 477 | 4 | 0.00839 |
| Folic Acid | 441 | 4 | 0.00907 |
| Niacinamide | 122 | 4 | 0.0328 |
| Pyridoxine hydrochloride | 204 | 4 | 0.0196 |
| Riboflavin | 376 | 0.4 | 0.00106 |
| Thiamine hydrochloride | 337 | 4 | 0.0119 |
| Vitamin B12 | 1355 | 0.0068 | 0.000005 |
| i-Inositol | 180 | 7.2 | 0.04 |

| **Inorganic Salts** | | | |
|---|---|---|---|
| Calcium Chloride (CaCl2) (anhyd.) | 111 | 200 | 1.8 |
| Ferric Nitrate (Fe(NO3)3"9H2O) | 404 | 0.1 | 0.000248 |
| Magnesium Chloride (anhydrous) | 95 | 77.3 | 0.814 |
| Potassium Chloride (KCl) | 75 | 400 | 5.33 |
| Sodium Bicarbonate (NaHCO3) | 84 | 2200 | 26.19 |
| Sodium Chloride (NaCl) | 58 | 3000 | 51.72 |
| Sodium Phosphate monobasic (NaH2PO4-H2O) | 138 | 125 | 0.906 |
| Zinc sulfate (ZnSO4-7H2O) | 288 | 0.194 | 0.000674 |

| **Other Components** | | | |
|---|---|---|---|
| D-Glucose (Dextrose) | 180 | 4500 | 25 |
| HEPES | 238 | 2600 | 10.92 |
| Sodium Pyruvate | 110 | 25 | 0.227 |

N2 supplement may be purchased from Invitrogen. The Invitrogen N2 supplement may comprise the following ingredients:

**Table 2: N2 Supplement**

| **Components** | **Molecular Weight** | **Concentration (mg/L)** | **mM** |
|---|---|---|---|
| **Proteins** | | | |
| Human transferrin (Holo) | 10000 | 10000 | 1 |
| Insulin recombinant full chain | 5807.7 | 500 | 0.0861 |

| **Other components** | | | |
|---|---|---|---|
| Progesterone | 314.47 | 0.63 | 0.002 |
| Putrescine | 161 | 1611 | 10.01 |
| selenite | 173 | 0.52 | 0.00301 |

For example, the medium may comprise about 1 to about 100, about 5 to about 50, about 10 to about 25 or about 20 ng/mL of EGF. The medium further comprises about 1 to about 100 ng/mL, about 5 to about 50, about 10 to about 25, or about 20 ng/mL of b-FGF. The N2 supplement may be present in the medium at a concentration of about 1×, and L-glutamine may be present in an amount of about 0.1 to about 10 mM, about 1 to about 5 mM, or about 1.3 to about 3 mM. The medium may further comprise a suitable amount of the candidate compound, for example from about 0.1 nM to about 10 mM, or 1 nM to about 1 mM.

In certain embodiments, the culturing medium is substantially free of serum or, preferably, completely free of serum. A culture medium substantially free of serum refers to medium having less than about 10% serum, more particularly less than about 2% or 0.1% serum; in certain embodiments, substantially free of serum refers to less than about 0.5% serum. A culture medium completely free of serum has 0% serum. While not being bound by any particular theory, it is believed serum may contain inconsistent and undetermined amounts of growth factors, which has the potential to impact the extent of neurogenesis. Accordingly, serum-free media eliminate the effects of serum on the extent of neurogenesis. Neurogenesis observed in ADSCs cultured in serum-free media can thus be attributed to the candidate compound rather than the presence of serum.

The aforementioned methods are useful in a rapid neuronal differentiation platform ("RNDP"). The RNDP may advantageously be used to quickly screen large numbers of potential neurogenesis modulating compounds. Compounds can be rapidly screened using multi-well plates and/or by testing several compounds at once or libraries of compounds for high through-put results. Compounds identified in the RNDP are further investigated using an extended platform, if desired.

An extended neuronal differentiation protocol ("ENDP") further comprises a priming step. The ENDP is useful to further investigate and confirm the results of an RNDP. While not being bound by any particular theory, it is believed that priming the ADSCs allows for improved neuronal morphology, thereby providing additional insight in the neurogenesis modulating potential of a given compound. Accordingly, in some embodiments, the ADSCs are primed prior to culturing in the presence of a candidate compound. For example, the ADSCs can be primed for about 1 to about 5 days in a suitable priming medium prior to culturing with the candidate compound. In other embodiments, the ADSCs are primed for about 1 to about 3 days, or for about 3 days.

In some embodiments, the priming medium comprises a neural basal medium (such as Neurobasal Medium™ from Invitrogen), with suitable concentrations of EGF, b-FGF, and N2 supplement. Suitable concentrations of EGF include about 1 to about 100 ng/mL, about 5 to about 50, about 10 to about 25 or about 20 ng/mL. Suitable concentrations of b-FGF include about 1 to about 100, about 5 to about 50, about 10 to about 25, or about 20 ng/mL of b-FGF. The N2 supplement may be present in the medium at a concentration of about 1×. The priming medium may be substantially free of serum or, more preferably, completely free of serum. A priming medium substantially free of serum refers to medium having less than about 10% serum, for example less than about 2% or 0.1% serum, while a culture medium completely free of serum has 0% serum. Furthermore, the priming medium may be free of or substantially free of the candidate compound.

In embodiments wherein the ADSCs are primed prior to being cultured in the presence of a candidate compound, the ADSCs are subsequently cultured in a suitable culture medium for about 1 to about 5 days. In other embodiments, the ADSCs are cultured for about 1 to about 3 days, or for about 3 days. After priming, the priming medium is removed and a culturing medium is added to the ADSCs. The culture medium comprises, for example, MesenPRO complete, available from Invitrogen. The culture medium may further comprise a suitable amount of the candidate compound, for example about 0.1nM to about 10 mM, or 1 nM to about 1 mM of the candidate compound. In a negative control experiment, the culture medium is free of or substantially free of the candidate compound. In a positive control experiment, the culture medium comprises a known neurogenesis promoting compound, such as DHA.

In some embodiments, the cultureware used to culture the ADSCs is coated with a unique combination of matrix proteins designed to mimic the *in vivo* environment of the central nervous system, maximize cellular neuronal differentiation activity, and enhance cellular attachment. In one embodiment, the coating comprises poly-L-ornithine and bovine plasma fibronectin. The coated cultureware can be prepared by contacting the cultureware with a solution of poly-L-ornithine and a solution of bovine fibronectin. The contacting steps may be performed in any order, simultaneously, or substantially simultaneously. For example, the cultureware can be contacted with the poly-L-ornithine prior to the bovine fibronectin or after the fibronectin. Alternatively, the poly-L-ornithine and bovine fibronectin are contacted with the cultureware simultaneously or substantially simultaneously.

Another aspect of the disclosure relates to an *in vitro* method of promoting neurogenesis in ADSCs comprising: culturing the ADSCs in the presence of a neurogenesis-promoting compound. Neuronal cells and neuron-like cells generated by the aforementioned methods may be maintained in culture, passaged, or cryopreserved. The method thus can provide human neuronal cells and neuron-like cells for use in the laboratory, such as for drug screening. In some embodiments, the method further comprises determining the extent of neurogenesis in the ADSCs, as described in the aforementioned screening methods.

Another aspect of the disclosure relates to a system for identifying a neurogenesis-modulating compound, comprising: ADSCs; cultureware comprising coating that mimics the central nervous system; and a culture medium. In some embodiments, the coating comprises bovine fibronectin and poly-L-ornithine. In systems useful in the RNDP, the culture medium the culture medium comprises a neural basal medium, EGF, b-FGF, N2 supplement, and L-glutamine. Systems useful in the ENDP, further comprise a priming medium, such as a medium comprising a neural basal medium, EGF, b-FGF, N2 supplement, and culture medium comprising MesenPRO Complete.

### EXAMPLES

### hADSCs

The hADSCs used in the following procedures are purchased from commercial resources and grown in the maintenance media consisting of Complete MesenPRO RS medium with supplement and L-glutamine. The subculture of hADSCs is performed when cell culture reaches confluence. To passage hADSCs, the following procedure is used: i) aspirate the Complete MesenPRO RS medium from the cells; ii) rinse the surface area of the cell layer with Dulbecco's phosphate buffered saline (DBPS) buffer by adding the DPBS to the side of the vessel opposite the attached cell layer and rocking the vessel back and forth several times; iii) remove the DPBS by aspiration and discard; iv) detach the cells by adding a sufficient volume of pre-warmed trypsin-EDTA solution without phenol red to cover the cell layer; v) incubate at 37°C. for approximately 7 minutes; vi) observe the cells under a microscope to determine if additional incubation is needed; vii) add 3mL of the maintenance media to the plate, mix the cell suspension, add the suspension to a 15mL centrifuge tube and centrifuge at 210g for 5 minutes; viii) determine the total number of cells and percent viability using a hemacytometer; ix) add Complete MesenPRO RS medium to each vessel so that the final culture volume is 0.2mL - 0.5mL per cm²; x) seed the cells by adding the appropriate volume of cells to each vessel and incubate at 37°C., 5% CO₂ and 90% humidity; and xi) three or four days after seeding, completely remove the medium and replace with an equal volume of Complete MesenPRO RS medium.

### Coating

Before seeding the passaged hADSCs on fresh culture plates, the surfaces of the cultureware are washed with sterile DPBS solution three times, followed by multiple rinses with sterile water. The first layer of coating is poly-L-ornithine. The coating is prepared by adding 0.1 mg/mL of poly-L-ornithine and incubating at 37°C. for one hour. The plate is washed three times with DPBS, 15 minutes per wash. The second layer of coating is bovine plasma fibronectin. The fibronectin is diluted in DPBS from stock to 1:1000 and 500 □L is added to each well. The plate is left at room temperature for one hour. One final wash with 500 □L per well of DPBS is performed and the plate is used immediately.

### Medium

hADSCs can be maintained in an undifferentiated state or guided to differentiate using different culture media. Certain culture media are capable of guiding ADSCs to differentiate into neuronal cells. Exemplary media are set forth in Tables 3, 4 and 5.

**Table 3.**

| ***Serum*-*free RNDP medium*** | |
|---|---|
| **component** | **Final concentration** |
| neural basal medium | 500 mL |
| EGF | 20 ng/mL |
| b-FGF | 20 ng /mL |
| N2 supplement | 1× |
| L-glutamine | 2 mM |

**Table 4.**

| ***Serum***-***free ENDP priming medium*** | |
|---|---|
| **component** | **Final concentration** |
| neural basal medium | 500 mL |
| EGF | 20 ng/mL |
| bFGF | 20 ng/mL |
| N2 supplement | 1× |

**Table 5.**

| ***ENDP differentiation medium*** | |
|---|---|
| **component** | **Final concentration** |
| MesenPRO complete | 500 mL |

### RNDP Protocol

Two independent screening protocols are described, designated as rapid neuronal differentiation platform (RNDP) and extended neuronal differentiation platform (ENDP). The RNDP protocol provides rapid screening of large numbers of candidate compounds in a relatively short period of time. RNDP allows the rapid identification of compounds that either promote or inhibit neurogenesis, or that have no effect on neurogenesis. The RNDP may be followed by an ENDP in order to further investigate and confirm the results.

The subculture media of the hADSCs described above is removed from the culture dish, and the dish is then gently washed with 5-10 mL of sterile DPBS. The DPBS is removed and 1.5 mL of trypsin-EDTA is added to completely cover the cell layer. The dish is placed back in the incubator for seven minutes. The plate is then gently tapped to detach cells completely, 3 mL of the maintenance media is added to the plate, and the cell suspension is mixed and added to 15 mL centrifuge tube. The desired cell density (1x10⁴ cells/well) is taken to another 15 mL tube and placed to centrifuge at 210g for 5 minutes. The cell pellet is resuspended in an appropriate volume of pre-warmed serum-free rapid neuronal differentiation medium as set forth in Table 1 and seeded onto each well of tissue culture plate. The candidate compounds for each well are added sequentially. The plate is put back into the incubator. The effects of the candidate compounds are quickly and easily observed using phase contrast microscopy images, which are usually taken once immediately before treatment, three hours post treatment and each day thereafter for three days. With a fast turnover time, the best results typically occur within 36 hours. After images are collected, data analysis and comparison is made to determine the effectiveness of each compound or mixture of compounds in modulating neurogenesis. Neuronal differentiation is determined by observing neuronal morphology. Some changes in the cells include shrinking of the cytoplasm, formation of axons and dendrite-like cytoplasmic projections. These changes begin with the cytoplasm of hADSCs retracting toward the nucleus to form contracted cell bodies with cytoplasmic extensions. Cells eventually develop a morphology that resembles bi-polar, tri-polar, and multi-polar neuronal cells.

### ENDP Protocol

The ENDP protocol provides a method for further investigation of the results of the RNDP and also allows additional time for priming the hADSCs for further differentiation into various neuronal cell lineages. While not being bound by any particular theory, the priming drives transdifferentiation of the hADSCs from mesoderm lineages to neural ectoderm.

The hADSCs are seeded on culture plates with coated surfaces and grown in the serum-free ENDP priming medium (see table 2) for at least 72 hours. The priming medium is removed and neuronal differentiation medium added (see Table 3) in the presence or absence of at least one candidate compound. The cultures are then incubated for an extended period of time for further neuronal development. After three days of incubation, the cells are examined under microscope for morphological changes. The percentage and length of neurites can be measured by using open software of Image J with an appropriate plug-in. The cells can further be studied for various neuronal markers to further confirm neuronal differentiation.

### Discovery of brain nutrients

The purpose of this investigation is to determine the neurogenesis effect of various nutrients (candidate compounds) using both RNDP and ENDP platforms. The candidate compounds are tested individually and compared to the positive control, docosahexaenoic acid (DHA), and the negative control. Pre-warmed serum-free medium contains Neural Basal medium with L-glutamine, 20ng/mL of b-FGF, 20ng/mL of EGF and N2 supplement. The candidate compound is added to individual wells at various concentrations in the serum-free medium. The candidate compounds are selected from the group consisting of ARA, EPA (cis-5,8,11,14,17-eicosapentaenoic acid) and resveratrol. Compounds are tested in varying concentrations, ranging in the nanomolar to micromolar range. The compounds are tested individually and compared to the positive control, docosahexaenoic acid (DHA), and the negative control. The experiments are repeated in triplicate. The nutrients found to promote neurogenesis or demonstrate use as a medicament are further screened in various combinations. These experiments are also repeated in triplicate.

The effects of the candidate compounds are easily and quickly observed under phase contrast microscopy for up to one week with images usually taken once immediately before treatment with the candidate compound, three hours post treatment, and each day thereafter for three days. With a fast turnover time, the best results typically occur within 36 hours. After images are collected, data analysis and comparison is made to determine the effectiveness of each compound or combination of compounds in promoting neurogenesis. Neuronal differentiation is determined by neuronal morphology. Some of these changes include shrinkage of the cytoplasm, and formation of axons and dendrite-like cytoplasmic projections (neurites). These changes begin with the cytoplasm of hADSCs retracting towards the nucleus to form contracted cell bodies with cytoplasmic extensions. Cells eventually develop a morphology that resembles bi-polar, tri-polar and multi-polar neuronal cells.

Of the above candidate compounds, resveratrol, ARA, EPA, cholesterol and DHA are examples of compounds that effectively promote neurogenesis. The test concentrations and effective concentrations are depicted in table 6:

**Table 6: Examples of compounds identified as effectively promoting neurogenesis**

| **Compound** | **Testing range** | **Effective range** |
|---|---|---|
| DHA | 1nM -1mM | 5-20 □M |
| ARA | 1nM -1mM | 2-10 □M |
| EPA (*Cis*-5,8,11,14,17-Eicosapentaenoic acid) | 1nM -1mM | 10-40 □M |
| Cholesterol | 1□M - 10□M | 50-200 □M |
| Resveratrol | 100nM -50mM | 2□□M -20 mM |

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The methods and compositions of the present disclosure, including components thereof, can comprise, consist of, or consist essentially of the essential elements and limitations of the embodiments described herein, as well as any additional or optional ingredients, components or limitations described herein.

As used herein, the term "about" should be construed to refer to both of the numbers specified in any range. Any reference to a range should be considered as providing support for any subset within that range.

## Claims

1. A method for identifying a neurogenesis-modulating compound, comprising:
culturing adipose-derived stem cells (ADSCs) in the presence of a candidate compound;
determining the extent of neurogenesis in the ADSCs;
culturing ADSCs in the presence of docosahexaenoic acid (DHA);
determining the extent of neurogenesis of the ADSCs cultured in the presence of DHA; and
comparing the extent of neurogenesis in the ADSCs cultured in the presence of the candidate compound to the extent of neurogenesis in the ADSCs cultured in the presence of DHA, wherein an increase in the extent of neurogenesis in the ADSCs cultured in the presence of the candidate compound compared to the extent of neurogenesis in the ADSCs cultured in the presence of DHA indicates that the candidate compound is a neurogenesis-promoting compound.

2. The method of claim 1, further comprising:
culturing ADSCs in the absence of the candidate compound,
determining the extent of neurogenesis in the ADSCs cultured in the absence of the candidate compound, and
comparing the extent of neurogenesis in the ADSCs cultured in the presence of the candidate compound to the extent of neurogenesis of the ADSCs cultured in the absence of the candidate compound.

3. The method of claim 2, wherein an increase in the extent of neurogenesis in the ADSCs cultured in the presence of the candidate compound compared to the extent of neurogenesis in the ADSCs cultured in the absence of the candidate compound indicates that the candidate compound is a neurogenesis-promoting compound.

4. The method of claim 2, wherein a decrease in the extent of neurogenesis in the ADSCs cultured in the presence of the candidate compound compared to the extent of neurogenesis in the ADSCs cultured in the absence of the candidate compound indicates that the candidate compound is a neurogenesis-inhibiting compound.

5. The method of claim 1, wherein the extent of neurogenesis is determined by observing a change in cell morphology of the ADSCs.

6. The method of claim 5, wherein the change in cell morphology is shrinkage of cell cytoplasm, formation of a neurite, formation a dendrite-like projection, formation of an axon, or a combination thereof.

7. The method of claim 5, wherein the change in cell morphology is observed by microscopy.

8. The method of claim 7, wherein the change in cell morphology is observed by contrast microscopy.

9. The method of claim 1, wherein the adipose-derived stem cells are human adiposederived stem cells (hADSCs).

10. The method of claim 1, wherein the ADS Cs are cultured in the presence of the candidate compound for 1 to 5 days.

11. The method of claim 1, wherein the ADSCs are cultured in a medium comprising a neural basal medium, EGF, b-FGF, N2 supplement, and L-glutamine.

12. The method of claim 1, further comprising priming the ADSCs for 1 to 5 days in a priming medium prior to culturing the cells in the presence of the candidate compound.

13. The method of claim 12, wherein the priming medium comprises a neural basal medium, EGF, b-FGF, and N2 supplement.

14. The method of claim 12, wherein the ADSCs are cultured in the presence of the candidate compound for 1 to 5 days.

## Patentansprüche

1. Verfahren zum Identifizieren einer neurogenesemodulierenden Verbindung, umfassend:
Kultivieren von aus Fettgewebe stammenden Stammzellen (ADSCs, engl. adipose-derived stem cells) in Gegenwart einer Kandidatenverbindung;
Bestimmen des Umfangs von Neurogenese in den ADSCs;
Kultivieren von ADSCs in Gegenwart von Docosahexaensäure (DHA);
Bestimmen des Umfangs von Neurogenese der ADSCs, kultiviert in Gegenwart von DHA; und
Vergleichen des Umfangs von Neurogenese in den ADSCs, kultiviert in Gegenwart der Kandidatenverbindung, mit dem Umfang von Neurogenese in den ADSCs, kultiviert in Gegenwart von DHA, wobei eine Zunahme in dem Umfang von Neurogenese in den ADSCs, kultiviert in Gegenwart der Kandidatenverbindung, verglichen mit dem Umfang von Neurogenese in den ADSCs, kultiviert in Gegenwart von DHA, anzeigt, dass die Kandidatenverbindung eine Neurogenese fördernde Verbindung ist.

2. Verfahren nach Anspruch 1, ferner umfassend:
Kultivieren von ADSCs in Abwesenheit der Kandidatenverbindung,
Bestimmen des Umfangs von Neurogenese in den ADSCs, kultiviert in Abwesenheit der Kandidatenverbindung, und
Vergleichen des Umfangs von Neurogenese in den ADSCs, kultiviert in Gegenwart der Kandidatenverbindung, mit dem Umfang von Neurogenese der ADSCs, kultiviert in Abwesenheit der Kandidatenverbindung.

3. Verfahren nach Anspruch 2, wobei eine Zunahme im Umfang von Neurogenese in den ADSCs, kultiviert in Gegenwart der Kandidatenverbindung, verglichen mit dem Umfang von Neurogenese in den ADSCs, kultiviert in Abwesenheit der Kandidatenverbindung, anzeigt, dass die Kandidatenverbindung eine Neurogenese fördernde Verbindung ist.

4. Verfahren nach Anspruch 2, wobei eine Abnahme im Umfang von Neurogenese in den ADSCs, kultiviert in Gegenwart der Kandidatenverbindung, verglichen mit dem Umfang von Neurogenese in den ADSCs, kultiviert in Abwesenheit der Kandidatenverbindung, anzeigt, dass die Kandidatenverbindung eine Neurogenese hemmende Verbindung ist.

5. Verfahren nach Anspruch 1, wobei der Umfang von Neurogenese durch Beobachtung einer Veränderung in Zellmorphologie der ADSCs bestimmt wird.

6. Verfahren nach Anspruch 5, wobei die Veränderung in Zellmorphologie ein Schrumpfen von Zellcytoplasma, Bildung eines Neuriten, Bildung eines dendritartigen Fortsatzes, Bildung eines Axons oder eine Kombination davon ist.

7. Verfahren nach Anspruch 5, wobei die Veränderung in Zellmorphologie mittels Mikroskopie beobachtet wird.

8. Verfahren nach Anspruch 7, wobei die Veränderung in Zellmorphologie mittels Kontrastmikroskopie beobachtet wird.

9. Verfahren nach Anspruch 1, wobei die aus Fettgewebe stammenden Stammzellen menschliche aus Fettgewebe stammende Stammzellen (hADSCs) sind.

10. Verfahren nach Anspruch 1, wobei die ADSCs in Gegenwart der Kandidatenverbindung für 1 bis 5 Tag(e) kultiviert werden.

11. Verfahren nach Anspruch 1, wobei die ADSCs in einem Medium kultiviert werden, umfassend ein neurales Basalmedium, EGF, b-FGF, N2-Zusatz und L-Glutamin.

12. Verfahren nach Anspruch 1, ferner umfassend Priming der ADSCs für 1 bis 5 Tag(e) in einem Priming-Medium vor dem Kultivieren der Zellen in Gegenwart der Kandidatenverbindung.

13. Verfahren nach Anspruch 12, wobei das Priming-Medium ein neurales Basalmedium, EGF, b-FGF und N2-Zusatz umfasst.

14. Verfahren nach Anspruch 12, wobei die ADSCs in Gegenwart der Kandidatenverbindung für 1 bis 5 Tag(e) kultiviert werden.

## Revendications

1. Un procédé pour identifier un composé de modulation de la neurogénèse, comprenant :
- la culture de cellules souches dérivées d'adipose (ADSCs) en présence d'un composé candidat ;
- la détermination de l'étendue de la neurogenèse dans les ADSCs;
- la culture des ADSC en présence d'acide docosahexaénoïque (DHA) ;
- la détermination de l'étendue de la neurogenèse des ADSCs cultivés en présence de DHA et
- la comparaison de l'étendue de la neurogenèse dans les ADSCs cultivés en présence du composé candidat à l'étendue de la neurogenèse dans les ADSCs cultivés en présence de DHA,
dans lequel procédé une augmentation de l'étendue de la neurogenèse dans les ADSC cultivés en présence du composé candidat comparée à l'étendue de la neurogenèse dans les ADSC cultivés en présence de DHA indique que le composé candidat est un composé promouvant la neurogénèse

2. Le procédé selon la revendication 1, comprenant en outre:
- la culture de cellules souches dérivées d'adipose (ADSCs) en l'absence d'un composé candidat;
- la détermination de l'étendue de la neurogenèse dans les ADSCs cultivés en l'absence du composé candidat, et
- la comparaison de l'étendue de la neurogenèse dans les ADSCs cultivés en présence du composé candidat à l'étendue de la neurogenèse des ADSCs cultivés en l'absence de du composé candidat,

3. Le procédé selon la revendication 2, dans lequel une augmentation de l'étendue de la neurogénèse dans les ADSCs cultivés en présence du composé candidat comparée à l'étendue de la neurogenèse dans les ADSCs cultivés en l'absence du composé candidat indique que le composé candidat est un composé promouvant la neurogénèse.

4. Le procédé selon la revendication 2, dans lequel une diminution de l'étendue de la neurogénèse dans les ADSCs cultivés en présence du composé candidat comparée à l'étendue de la neurogenèse dans les ADSCs cultivés en l'absence du composé candidat indique que le composé candidat est un composé inhibant la neurogénèse

5. Le procédé selon la revendication 1, dans lequel l'étendue de la neurogenèse est déterminée en observant un changement de la morphologie cellulaire des ADSCs.

6. Le procédé selon la revendication 5, dans lequel le changement de morphologie cellulaire est le rétrécissement du cytoplasme cellulaire, la formation d'un neurite, la formation d'une projection en forme d'une dendrite, la formation d'un axone, ou une combinaison de ceux-ci.

7. Le procédé selon la revendication 5, dans lequel le changement de morphologie cellulaire est observé par microscopie.

8. Le procédé selon la revendication 7, dans lequel le changement de morphologie cellulaire est observé par microscopie contrastée.

9. Le procédé selon la revendication 1, dans lequel les cellules souches dérivées d'adipose sont des cellules souches dérivées d'adipose humain (hADSCs).

10. Le procédé selon la revendication 1, dans lequel les ADSCs sont cultivés en présence du composé candidat pendant 1 à 5 jours.

11. Le procédé selon la revendication 1, dans lequel les ADSCs sont cultivés dans un milieu comprenant un milieu basal neural, EGF, b-FGF, supplément de N2 et L-glutamine.

12. Le procédé selon la revendication 1, comprenant en outre l'amorçage des ADSCs pendant 1 à 5 jours dans un milieu d'amorçage préalablement à la culture des cellules en présence du composé candidat.

13. Le procédé selon la revendication 12, dans lequel le milieu d'amorçage comprend un milieu basal neural, EGF, b-FGF et supplément de N2.

14. Le procédé selon la revendication 12, dans lequel les ADSCs sont cultivés en présence du composé candidat pendant 1 à 5 jours.
